Europäisches Patentamt

European Patent Office

Office européen des brevets

(11) Publication number: **0 239 174 B1**

# EUROPEAN PATENT SPECIFICATION

(45) Date of publication of patent specification: **27.05.92**    (51) Int. Cl.⁵: **G01N 33/52**, G01N 33/53

(21) Application number: **87200535.0**

(22) Date of filing: **24.03.87**

(54) **Biological diagnostic device.**

(30) Priority: **25.03.86 US 843766**

(43) Date of publication of application:
**30.09.87 Bulletin  87/40**

(45) Publication of the grant of the patent:
**27.05.92 Bulletin  92/22**

(84) Designated Contracting States:
**AT BE CH DE ES FR GB IT LI LU NL SE**

(56) References cited:
**EP-A- 0 014 797          EP-A- 0 023 156**
**EP-A- 0 034 049          EP-A- 0 057 110**
**EP-A- 0 182 373          US-A- 3 723 064**
**US-A- 4 256 693**

(73) Proprietor: **PB Diagnostic Systems, Inc.**
**750 Main Street**
**Cambridge, MA 02139(US)**

(72) Inventor: **Grenner, Gerd**
**326 Hemlock Circle**
**Lincoln, MA 01773(US)**
Inventor: **Hachmann, Jurgen H.**
**1 Village Circle**
**Lexington, MA 02173(US)**
Inventor: **Manning, James J.**
**19 Holmes Street**

**Braintree, MA 02184(US)**
Inventor: **Pauly, Hans Erwin**
**Finkenstr. 1**
**W-3563 Buchenau(DE)**
Inventor: **Neely, George O.**
**414 Sudbury Street**
**Marboro, MA 01752(US)**
Inventor: **Staedter, Melitta**
**244 Kennedy Drive, Apt. 610**
**Malden, MA 02148(US)**

(74) Representative: **Smulders, Theodorus A.H.J.,**
**Ir. et al**
**Vereenigde Octrooibureaux Nieuwe Parklaan**
**97**
**NL-2587 BN 's-Gravenhage(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

## BACKGROUND OF THE INVENTION

Assay elements for the rapid analysis of analytes present in biological fluids are known in the art. Of particular interest are those which are capable of performing the analysis on samples of whole blood since these avoid the need for prior separation of blood cells from plasma such as by centrifuging. In such assay elements the sample, e.g., a drop of whole blood is applied to the element which includes some means for separating the cells (erythrocytes, leucocytes) from the plasma and the plasma, which includes the analyte of interest, then migrates to a reagent layer or layers. As a result of the interaction between the analyte and the reagent(s) present a detectable change is brought about in the element which corresponds to the analyte of interest. The detectable change can be a color change which may be evaluated visually or read spectrophotometrically such as with a densitometer. In another scheme based on the presence of fluorescent labelled biological species a fluorescent output signal can be generated and read spectrofluorometrically. In order to obtain accurate and reproducible results with machine readable diagnostic devices it is essential that the plasma or serum be distributed uniformly throughout the assay element so that a uniform signal or color is provided for reading by the instrument.

Various techniques for accomplishing the separation of the cells and uniform distribution of the plasma have been suggested in the art. U.S. Patent 3,216,804 disclosed an automatic chemical analyzer and sample dispenser and teaches that a uniform sample spot may be obtained by applying a drop of the sample on a filter paper which has fibers extending randomly in all directions or by using porous tapes or membranes. U.S. 3,607,093 discloses a device for testing biological fluids which comprises a liquid permeable membrane of uniform chemical composition which has substantially uniform porosity throughout. U.S. 3,723,064 discloses a multilayer device which has a sample receiving layer having uniform porosity which allows capillary migration to provide an even distribution of the components in the fluid. In cases where an incubation period is required, evaporation from the sample receiving layer could occur with a resultant change in the concentration of analyte in the sample.

The known techniques for filtering a whole blood sample and uniformly distributing the plasma have not been entirely satisfactory. In addition to the separation and distribution functions the sample application layer must satisfy a number of other requirements. For example, there must not be any significant amount of binding of the analytes and reagents to the material in the sample application layer, the plasma-analyte concentration level must not be affected, there should be no lysis of the blood cells and the layer must provide a metered amount of plasma to the underlying reagent layers. The known sample application layers and materials fail to provide one or more of these requisite functions.

In an effort to obtain a satisfactory sample application scheme it has been suggested to divide the filtering and distribution functions between different materials. U.S. Patent 4,477,575 discloses a technique for separating cells from plasma or serum which involves applying a sample of whole blood to a layer of glass fibres having an average diameter of 0.2 to 5 $\mu$m and a density of 0.1 to 0.5 g/cm$^3$. There are also disclosed various biological diagnostic devices which incorporate such a glass fiber layer. In one embodiment (see, for example, Fig. 11) the plasma or serum which passes through the filter layer is taken up by a layer of an absorbent material such as cellulose paper or a synthetic fiber fleece which is in contact with the reaction layer. Due to capillary forces the plasma or serum is passed into the reaction layer where the detection reaction takes place.

This arrangement is not satisfactory in all instances. For example, it is not suitable for use with thin film multilayer diagnostic test elements. In such thin film multilayer elements the volume of fluid which is supplied to the test element must be very small and very precisely metered. Since the paper or fiber fleece is relatively thick and has a relatively large surface area the volume of fluid supplied to the test element is relatively large and the precision with which the amount of fluid can be controlled is relatively lower. In addition, because of the area of the relatively thick absorbent material it may give rise to relatively high levels of nonspecific binding of the analyte.

European Patent Application 0 160 916 discloses, in an analytical element, a volume filtration layer consisting of a fibrous material and a spreading layer having a liquid retaining capacity which is larger than that of the volume filtration layer. The spreading layer may be a fibrous material, woven cloth, knitted cloth or a non-fibrous porous medium. This arrangement suffers from various of the disadvantages previously discussed. For example, when the spreading layer is a non-fibrous membrane filter the pores of the membrane material are very small and fluid will not pass through easily without the application of pressure.

Accordingly, there is a continuing need for biological diagnostic devices having sample application units which can efficiently and effectively remove from a sample of a biological fluid any components which could interfere with the assay to be performed and provide plasma or serum to the test element without affecting the accuracy of the analysis.

## SUMMARY OF THE INVENTION

It is therefore an object of this invention to provide a novel biological diagnostic system.

It is another object of the invention to provide a diagnostic device which includes a filter element and a fluid delivery element which is in fluid contact with a diagnostic test element.

It is still another object to provide a diagnostic device wherein the fluid delivery element comprises a layer which has a plurality of grooves in the surface thereof which is adjacent the filter element to collect fluid which passes through the filter element.

It is a further object to provide a diagnostic device wherein the filter element is a fibrous element which is capable of separating cells from plasma or serum.

Yet another object is to provide a diagnostic device which is adapted to be used with samples of whole blood.

Still another object is to provide a process for analyzing for an analyte of interest in a sample of a biological fluid.

## BRIEF SUMMARY OF THE INVENTION

These and other objects and advantages are accomplished in accordance with the invention by providing a biological diagnostic device and process for rapidly, efficiently and accurately analyzing a biological fluid. The diagnostic device comprises a diagnostic test element and a sample application unit comprising a filter element and a fluid delivery element which is in fluid contact with the diagnostic test element. The fluid delivery element comprises a layer which includes a plurality of grooves, in the surface thereof adjacent the filter element. The fluid which passes through the filter is collected in the grooves of the delivery element and subsequently delivered to the diagnostic test element. The diagnostic test element is arranged on, i.e., in contact with, the grooved surface of the delivery element.

In operation, a sample of a biological fluid, which in a preferred embodiment is whole blood but which may be any biological fluid such as, for example, a cell culture fluid, is applied to the filter element. The filter element may comprise any suitable filter material, synthetic or naturally occurring, which is capable of removing from the fluid sample the components thereof which could interefere with the analysis. The fluid which passes through the filter element is collected by the grooves in the surface of the fluid delivery element and is subsequently brought to the diagnostic test element where it is imbibed into that element. In this manner there is obtained a uniform distribution of the fluid throughout the area of the test element surface which will be analyzed. It should be noted here that the detectable change in the test element, whether it is a color change which is to be evaluated visually or read out spectrophotometrically or whether it is some other type of change such as the generation of a fluorescent output signal which is to be read out spectrofluorometrically, will be analyzed over a specific portion of the test element surface typically a circular or rectangular area in the center of the test element. Thus, it is essential to obtain a uniform distribution of the test fluid throughout the area of the test element which will be analyzed.

In a preferred embodiment the diagnostic test element is a thin film multilayer test element. The sample delivery element is particularly well suited for use with thin film multilayer diagnostic test elements because the volume in the grooves can be made very small and controlled very precisely. For thin film multilayer diagnostic test elements it is necessary to deliver a relatively small volume of fluid to the test element. To ensure that the test element receives a volume of fluid equivalent to its wet uptake capacity, the fluid delivery system typically should be capable of delivering from about 110% to about 200% of the wet uptake volume of the test element. This requirement can be met by the groove delivery element because as noted above, the volume of the grooves can be made relatively small. Accordingly, the delivery element is capable of providing, as is required with thin film multilayer test elements, a small volume of precisely metered sample fluid. A further advantage of the biological diagnostic device of the invention is that the fluid which passes through the filter element is not exposed to the ambient environment very much, or at all, prior to being delivered to the diagnostic test element. Thus, any evaporation of any significance which could lead to a change in the analyte concentration is prevented.

## BRIEF DESCRIPTION OF THE DRAWINGS

For a better understanding of the invention as well as other objects and further features thereof, reference is made to the following detailed description of various preferred embodiments thereof taken in conjunction with the accompanying drawings wherein;

Fig. 1 is a partially schematic, perspective view of one embodiment of a diagnostic device according to the invention;

Fig. 2 is a partially schematic perspective view of a fluid delivery element;

Fig. 3 is a partially schematic, top view of one embodiment of a fluid delivery element;

Fig. 4 is a partially schematic, cross-sectional view of another embodiment of a diagnostic device according to the invention.

Fig. 5 is a partially schematic, cross-sectional view of another embodiment of a diagnostic device according to the invention;

Fig. 6 is a partially schematic, cross-sectional view of another embodiment of a diagnostic device according to the invention; and

Fig. 7 is a partially schematic, top view of another embodiment of a diagnostic device according to the invention.

## DESCRIPTION OF THE PREFERRED EMBODIMENTS

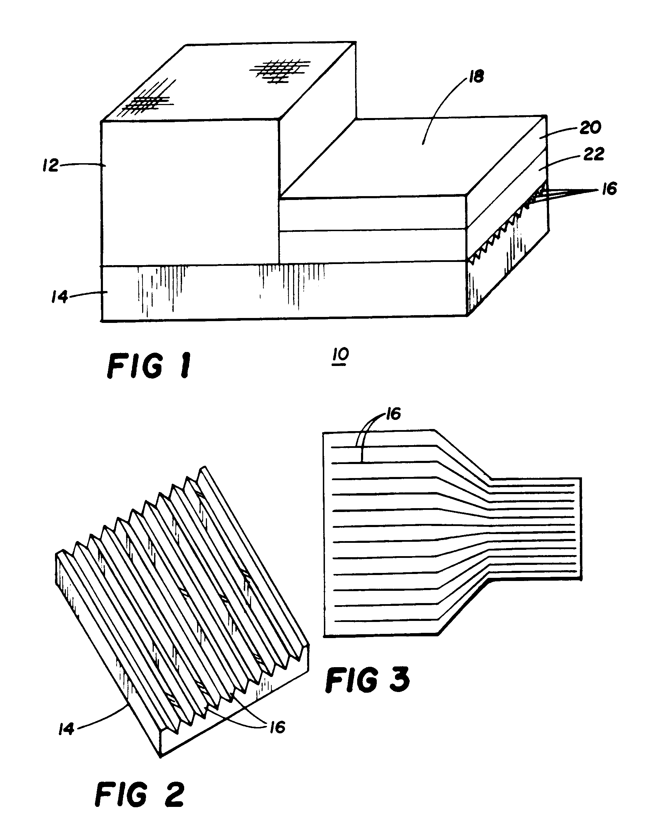

Referring to Fig. 1 there is seen a preferred embodiment of a diagnostic element 10 according to the invention. It should be recognized that the thickness of the device has been magnified for ease of illustration; the actual preferred devices of the invention are relatively thin, having typical thicknesses in the range of from about 1 mm to about 3 mm. The diagnostic device 10 comprises a filter element 12, a fluid delivery element 14 having a plurality of grooves 16 in the surface thereof which is adjacent the filter element and the diagnostic test element generally designated 18.

As noted previously, the filter element 12 may be any suitable material, synthetic or naturally occurring or a mixture of each type, which is capable of removing from the fluid sample any component(s) which could interefere with the analysis and which is inert to the analyte(s) of interest, that is, will not prevent any significant amount of said analyte(s) from passing through the filter element whether because of adsorption, reaction or otherwise. As shown the filter element is a flat, sheet-like layer; however, the element may be provided in any desired shape or configuration such as a pad or a curved layer. The type of filter material employed in the device is dependent upon the type of biological fluid to be analyzed. For example, a microporous filter element may be used to remove bacteria cells or microorganisms from the sample fluid. In a preferred embodiment wherein the sample is whole blood the filter element comprises fibrous material which is capable of separating cells such as, for example, erythrocytes, leucocytes, etc. from plasma or serum. Typical suitable fibrous materials include glass, quartz, cellulose acetate, cellulose, synthetic polymer fibers, such as polyamides or polyesters, and the like. In a preferred embodiment the fibrous material may be treated with a material such as gelatin, either inert or deionized, or serum albumin to substantially reduce or eliminate any binding thereto by an analyte of interest. The fibrous filter element typically has an average thickness of from about 0.5 mm to about 2.0 mm. The filter element 12 may be impregnated with a material which is capable of removing specific components from the fluid sample, for example, lipoproteins. Titanium dioxide is suitable for this purpose. Antibodies specific to components in the fluid may also be used.

The fluid delivery element 14 may comprise a sheet of any suitable material, transparent or opaque, including synthetic, film-forming polymeric materials, e.g., polyvinylacetate, polyvinylchloride, polyvinylchloride-polyvinylalcohol copolymers, polypropylene, polystyrene, cellulose acetate butyrate, hydrolyzed cellulose acetate butyrate and the like; metals, ceramics, etc. The material should be non-absorbent or substantially nonabsorbent with respect to the fluid or any of the components thereof. In a preferred embodiment the grooved surface of the delivery element is treated such as by hydrolysis or with a material which causes its surface to be more easily wetted by the fluid. Consequently the fluid can be delivered more rapidly to the test element. Proteins such as gelatins and albumins as well as surfactants are suitable for this purpose. Some metals and polymeric materials strongly absorb proteins and the contact angles of fluids applied thereto are changed significantly. As noted above, the volume of the grooves can be relatively small. The small surface of the fluid delivery element is advantageous since any nonspecific binding of the analyte of interest to the delivery element is thereby minimized. Hydrolyzed cellulose acetate butyrate is a preferred material for the fluid delivery element because it is highly wettable. In a preferred embodiment the fluid delivery element comprises a clear polymeric material which allows an output signal, such as a spectrophotometric signal, to be read out through the delivery element. The thickness of the fluid delivery element is typically about 1 mm.

The grooves 16 in the fluid delivery element may be of any shape such as, for example, convex, concave, v-shaped or rectangular. The rectangular shaped grooves include those which are relatively wide and which are separated by relatively thin walls. Fig. 2 illustrates a delivery element wherein the grooves have a triangular shape. The number of grooves in the element is typically from about 4 to about 50 per cm. The groove depth is typically from about 0.025 to about 0.2 mm and preferably from about 0.05 to about 0.160 mm. The groove depth, number of grooves and the dimensions of the delivery element are dependent principally upon the amount of sample which is to be delivered to the diagnostic test element. For a device intended for use with whole blood sample, the groove depth is typically from about 0.1 to about 0.125 mm and typically the fluid delivery element includes from about 20 to about 40 grooves per cm with a void volume of from about 5-10 $\mu\ell/cm^2$.

The grooves may be parallel to each other and have uniform width and depth as illustrated in Fig. 2. In other embodiments the grooves may not be parallel to each other and the depth and/or the width may not be the same along their length . Fig. 3 illustrates a delivery element for a diagnostic device wherein the filter element is larger than the diagnostic test element. As can be seen some of the grooves 16 would be configured differently from the others to permit collection of the fluid passing through the filter element and delivery to the test element. Further, the grooves could be arranged in various shapes such as curvilinear, concentric, etc. The grooves can be made by various techniques such as embossing, laser etching, etc.

The diagnostic test element 18 as shown comprises a thin film multilayer structure with a support layer 20 and reagent layer 22. A typical thin film test element has a thickness of from about 0.1 mm to about 0.3 mm. The diagnostic device may incorporate any diagnostic test element whether a single layer or multilayer. The test element 18 is deposited on the grooved surface of the fluid delivery element such as by pressing it into contact or it can be adhered around its periphery to the delivery element with an adhesive material. The test element may be in contact with the filter element 12 as shown or it may be spaced apart from it. Where the filter and test elements are in contact with each other a thin film of a barrier material may be disposed at their interface to prevent any fluid from being drawn directly into the test element from the filter.

The diagnostic test elements which are useful in the diagnostic device of the invention are typically swellable when wet with fluid and the rate of swelling should be the same for a particular assay in all instances so as to give accurate, reproducible results. The pressure of the test element on the surface of the fluid delivery element may affect the rate of swelling of the former. In addition, the swelling of the test element may affect the rate at which the fluid is delivered to it. Accordingly, the grooves shape and depth and the number of grooves in the fluid delivery element should be selected so as to ensure that the grooves are not filled by the swelled surface of the test element to the extent that the rate of delivery of the fluid is significantly altered or that the delivery of the fluid is prevented. An inert, nonswellable porous layer may be included between the fluid delivery and assay elements to prevent blockage of the grooves by the swelled surface of the assay element. The inert, nonswellable porous layer may be provided as an integral part of the assay element or the fluid delivery element or as a discrete layer arranged between the two elements. A suitable porous layer comprises a layer of particulate material.

As noted previously, the delivery element 14 may be transparent or opaque. Further, after the reaction is completed the test element may be analyzed visually or by an instrument and this may be done while the test element remains as an integral part of the diagnostic device or it may be detached from the device for this purpose. In a preferred embodiment the device is read out spectrophotometrically or spectrofluorometrically with the test element included. Thus, in this case, either the fluid delivery element or the base of the test element or both would be transparent.

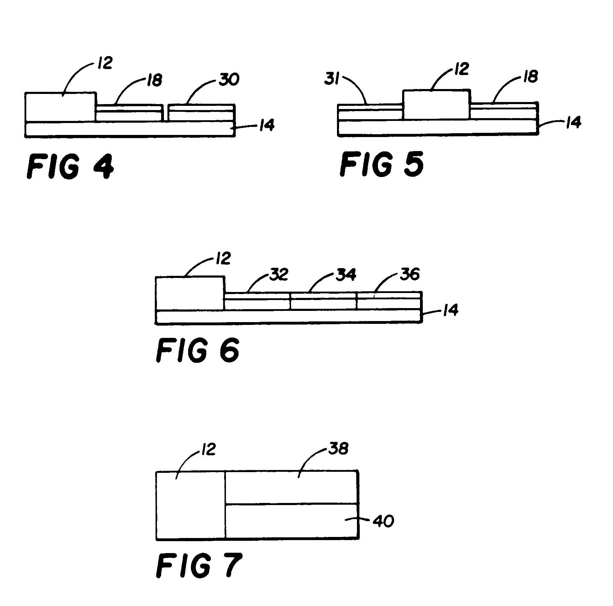

In another preferred embodiment illustrated in Fig. 4 a reagent blank element 30 is included in the diagnostic device. In a diagnostic element wherein the concentration of the desired analyte is determined by measuring a fluorescent output signal the reagent blank could test the fluorescence of the other materials present in the test element. In another embodiment a plurality of biological diagnostic test elements are disposed on the plasma or serum delivery element with each test element measuring the concentration of a different analyte present in the sample. The additional test elements or the reagent blank can be located in contact with or spaced apart from diagnostic test element 18 or they may be disposed on the other side of the filter element 12. Fig. 5 illustrates a diagnostic device wherein test elements 18 and 31 respectively are located on opposite sides of the filter element 12. Fig. 6 illustrates a diagnostic device having three diagnostic test elements 32, 34 and 36. Fig. 7 illustrates a diagnostic device wherein diagnostic test elements 38 and 40 respectively are arranged on the fluid delivery element in side by side fashion.

In commercial use the diagnostic test devices of the invention typically would be used with an automated test apparatus which would perform the analysis automatically and record the result. In such a test apparatus the diagnostic test device would typically be mounted in a holder which could be an integral part of the apparatus.

The invention will now be described further in detail with respect to specific preferred embodiments by way of examples it being understood that these are intended to be illustrative only.

EXAMPLE I

An experiment was conducted using 8 square filter pads of three different glass fiber materials and a clear plastic delivery element having approximately 21 convex, 0.16 mm deep, grooves per cm. The filter pad was placed at one end of the grooved surface of the delivery element and the remainder of the delivery element was covered with a 9 × 75 mm clear layer of polyester film base so the delivery element could be observed visually. A sample of whole blood was dropped onto the filter pad and the times required for the pad to fill were recorded. In addition the amount of plasma which was separated from the sample and entered the grooves of the delivery element was calculated. The grooves were measured to hold a volume of 5.8 $\mu\ell/cm^2$.

The experiment was conducted with glass fibers that were untreated; those which had been treated with deionized gelatin (having 12 ppm of calcium); and with those which had been treated with deionized gelatin and Tween 20 a surfactant available from Rohm and Haas Co. The glass fibers were treated with deionized gelatin by imbibing a 1% aqueous gelatin solution into the filter pad and subsequently washing three time with water. Where the glass fibers were also treated with Tween 20 in addition to gelatin, the first washing step was carried out with a 1% aqueous solution of Tween 20 followed by two washes with water.

| FILTER (treatment) | BLOOD VOL. [μℓ] | PAD FILLING TIME* [sec] | PLASMA YIELD* [μℓ] | OBSERVATIONS | |
|---|---|---|---|---|---|
| Sartorius 13430 (None) | 100 | 150 | 2 | + | |
| | 110 | 165 | 13.5 | + | + |
| | 120 | 93 | 19.7 | − | + |
| (Gelatin) | 100 | 105 | 7 | + | + |
| | 110 | 68 | 15.5 | − | + |
| | 120 | 60 | 22.5 | − | − |
| (Gelatin/Tween) | 100 | 20 | 4 | − | + |
| | 110 | 10 | 10 | + | − |
| | 120 | 10 | 22 | − | − |
| Whatman QM-A (None) | 60 | 25 | 14.5 | + | + |
| | 70 | 20 | 22 | − | + |
| | 80 | 18 | 29.5 | | |
| (Gelatin) | 60 | 17.5 | 10 | − | − |
| | 70 | 20 | 24 | − | − |
| | 80 | 17.5 | 30.5 | − | − |
| (Gelatin/Tween) | 60 | 17.5 | 15.5 | + | + |
| | 70 | 12.5 | 25 | − | − |
| | 80 | 10 | 29.5 | − | − |

EP 0 239 174 B1

| FILTER (treatment) | BLOOD VOL. [μl] | PAD FILLING TIME* [sec] | PLASMA YIELD* [μl] | OBSERVATIONS | |
|---|---|---|---|---|---|
| Whatman GF/B (None) | 80 | 210 | 4 | + | + |
| | 90 | 250 | 8 | - | + |
| | 100 | 270 | 8 | + | + |
| (Gelatin) | 80 | 120 | 10 | + | + |
| | 90 | 97.5 | 9.5 | + | + |
| | 100 | 65 | 13.5 | | + |
| (Gelatin/Tween) | 80 | 150 | 5 | + | + |
| | 90 | 95 | 10.5 | + | + |
| | 100 | 70 | 23.5 | + | + |

\* values are average of two determinations unless otherwise indicated by number of observations.

\+ indicates clear plasma in the grooves.

\- indicates some erythrocytes were observed in at least one groove.

It can be seen that the filter pads were generally effective in separating the plasma from the erthrocytes in the sample.

EXAMPLE II

An experiment was conducted to determine the extent of binding of four analytes, namely digoxin, hCG theophylline and insulin, to various glass fibers. The experiment was conducted with untreated glass fibers and those which had been treated with deionized gelatin and Tween 20. The filter treatments were carried out as described in Example I.

The analytes were labelled with Iodine - 125 and their concentrations in the plasma samples were:

7.81 $\mu$g digoxin/$\ell$ plasma

1 I.U. hCG/$\ell$ plasma

1.8 mg theophylline/$\ell$ plasma

50 $\mu$g insulin/$\ell$ plasma

A sample containing the analyte in mixed plasma was applied to an 0.8 cm diameter filter pad and allowed to incubate for 10 minutes at room temperature. The void volume for each filter pad was determined and the sample applied was equal to the void volume. For the Sartorius 13430 filter pad the sample was 90 $\mu\ell$; for the Whatman GF/B it was 65 $\mu\ell$; and for the Whatman QM-A, 45 $\mu\ell$. After the incubation period the filter pad was washed twice with 1 ml volumes of saline solution. Subsequently the radioactivity of the filter pad was measured and the percentage of nonspecific binding of the analyte was calculated.

The three types of glass fibers exhibited low nonspecific binding to the analytes: 2.6% of digoxin and 6.0% of insulin, respectively, were bound to the untreated Whatman GF/B filter material and 2.9% of digoxin and 6.2% of insulin, respectively were bound to the Whatman QM-A filter material. The percentage of nonspecific binding of the other analyte/filter combinations was less than 1%. Further, for the Whatman glass fiber materials it was found that treatment with deionized gelatin and Tween 20 reduced the nonspecific binding of digoxin and insulin to less than 1%.

EXAMPLE III

An experiment was conducted to determine the precision of fluid uptake for a test element of a diagnostic device according to the invention. The experiment was conducted with 7 × 7 mm square filter pads of two different glass fiber materials and a fluid delivery element having 32 convex, 0.125 mm deep grooves per cm. The filter pad was placed on one end of the grooved surface of the delivery element and a 7 × 7 mm square test element covered part of the remaining surface of the delivery element. The test element comprised a clear polystyrene base coated with an 18 g/m$^2$ agarose layer with the agarose layer in contact with the grooved surface of the fluid delivery element.

A sample of whole blood or plasma which was derived from the same blood sample by centrifugation was applied to the filter pad. After three minutes the test element was removed from the delivery element and tested for the uptake of fluid by weighing it.

The experiment was conducted with two different glass fiber filters which were treated with gelatin and Tween 20 as described in Example I.

| FILTER | SAMPLE | VOL. [μℓ] | NO. OF EXP. | FLUID UPTAKE* [mg/cm$^2$] |
|---|---|---|---|---|
| Sartorius 13430 | Whole Blood | 90 | 8 | 4.66 $\pm$ 0.08 |
| " | Plasma | 90 | 8 | 4.66 $\pm$ 0.18 |
| Whatman QM-A | Whole Blood | 50 | 8 | 4.40 $\pm$ 0.12 |
| " | Plasma | 50 | 8 | 4.58 $\pm$ 0.12 |

*mean value and standard deviation of 8 determinations

It can be seen that the fluid uptake of the test element was very precise for both the whole blood and plasma samples. Further, the fluid uptake of the Sartorius 13430 filter was the same for the whole blood and plasma samples and virtually the same with the Whatman QM-A filter.

**Claims**

1. A biological diagnostic device comprising a sample application unit including a fluid delivery element and a filter element arranged in fluid contact with the fluid delivery element; and at least one diagnostic test element arranged in such a manner that it is capable of receiving fluid delivered by the fluid delivery element, characterized in that the fluid delivery element has a plurality of grooves in a surface thereof for providing a path for fluids to flow therealong, the filter element is arranged in fluid contact with the grooves of the fluid delivery element; and at least one diagnostic test element is arranged in contact with the said surface of the fluid delivery element to receive fluid admitted to the grooves from the filter element.

2. The diagnostic device as defined in claim 1 wherein said filter element comprises a layer of fibrous material and is capable of separating cells from plasma or serum.

3. The diagnostic device as defined in claim 2 wherein said diagnostic test element is a multilayer immunoassay element.

4. The diagnostic device as defined in claim 2 wherein said fibrous filter material has been treated with deionized gelatin or albumin.

5. The diagnostic device as defined in claim 2 wherein said fibrous filter material has been treated with a surfactant.

6. The diagnostic device as defined in claim 2 wherein said fibrous filter material has been treated with a protein.

7. The diagnostic device as defined in claim 1 wherein said fluid delivery element includes from about 4 to about 50 grooves per cm, said grooves having a depth of from about 0.05 to about 0.160 mm.

8. The diagnostic device as defined in claim 7 wherein said fluid delivery element comprises a substantially transparent polymeric material.

**9.** The diagnostic device as defined in claim 8 wherein said fluid delivery element comprises hydrolyzed cellulose acetate butyrate.

**10.** The diagnostic device as defined in claim 1 comprising a plurality of diagnostic test elements.

**Revendications**

**1.** Dispositif pour le diagnostic biologique comportant une unité d'application d'échantillon comprenant une élément de délivrance de fluide et un élément de filtrage disposé en contact de fluide avec l'élément de délivrance de fluide; et au moins un élément de test de diagnostic disposé de façon à être capable de recevoir le fluide délivré par l'élément de délivrance de fluide, caractérisé en ce que l'élément de délivrance de fluide a une pluralité de rainures dans une surface de celui-ci pour procurer un chemin pour que les fluides coulent le long de celui-ci, en ce que l'élément de filtrage est disposé en contact de fluide avec les rainures de l'élément de délivrance de fluide; et en ce qu'au moins un élément de test de diagnostic est disposé au contact de ladite surface de l'élément de délivrance de fluide pour recevoir le fluide admis dans les rainures depuis l'élément de filtrage.

**2.** Dispositif de diagnostic selon la revendication 1, dans lequel ledit élément de filtrage comporte une couche de matériau fibreux et est capable de séparer les cellules du plasma ou du sérum.

**3.** Dispositif de diagnostic selon la revendication 2, dans lequel ledit élément de test de diagnostic est un élément multicouche pour immunotest.

**4.** Dispositif de diagnostic selon la revendication 2, dans lequel ledit matériau de filtrage fibreux a été traité avec de l'albumine ou de la gélatine désionisée.

**5.** Dispositif de diagnostic selon la revendication 2, dans lequel ledit matériau de filtrage fibreux a été traité avec un agent tensio-actif.

**6.** Dispositif de diagnostic selon la revendication 2, dans lequel ledit matériau de filtrage fibreux a été traité avec une protéine.

**7.** Dispositif de diagnostic selon la revendication 1, dans lequel ledit élément de délivrance de fluide comporte entre 4 et 50 rainures par cm environ, lesdites rainures ayant une profondeur comprise entre 0,05 et 0,160 mm environ.

**8.** Dispositif de diagnostic selon la revendication 7, dans lequel ledit élément de délivrance de fluide comporte un matériau polymère substantiellement transparent.

**9.** Dispositif de diagnostic selon la revendication 8, dans lequel ledit élément de délivrance de fluide comporte de l'acétobutyrate de cellulose hydrolisé.

**10.** Dispositif de diagnostic selon la revendication 1, comportant une pluralité d'éléments de test de diagnostic.

**Patentansprüche**

**1.** Biologisch-Diagnostische Vorrichtung, enthaltend eine Einheit zum Aufbringen einer Probe, mit einem Flüssigkeitsabgabe-Element und einem Filterelement, das mit dem Flüssigkeitsabgabe-Element in Flüssigkeitskontakt steht; und mindestens ein Diagnosetest-Element, das so angeordnet ist, daß es die von dem Flüssigkeitsabgabe-Element abgegebene Flüssigkeit aufnehmen kann, dadurch gekennzeichnet, daß das Flüssigkeitsabgabe-Element mehrere Rillen in einer seiner Oberflächen hat, die einen Weg für darin fließende Flüssigkeiten darstellen, wobei das Filterelement mit den Rillen des Flüssigkeitsabgabe-Elements in Flüssigkeitskontakt steht; und daß mindestens ein Diagnosetest-Element mit der Oberfläche des Flüssigkeitsabgabe-Elements in Berührung steht, um Flüssigkeit aufzunehmen, die den Rillen aus dem Filterelement zugeführt wird.

**2.** Diagnostische Vorrichtung nach Anspruch 1 , worin das Filterelement eine Schicht von faserartigem Material darstellt, das in der Lage ist, Zellen von Plasma oder von Serum zu trennen.

3.  Diagnostische Vorrichtung nach Anspruch 2, worin das Diagnosetest-Element ein mehrschichtiges Immunoassay-Element darstellt.

4.  Diagnostische Vorrichtung nach Anspruch 2, worin das faserartige Filtermaterial mit entionisierter Gelantine oder Albumin behandelt worden ist.

5.  Diagnostische Vorrichtung nach Anspruch 2, worin das faserartige Filtermaterial mit einem oberflächenaktiven Mittel behandelt worden ist.

6.  Diagnostische Vorrichtung nach Anspruch 2, worin das faserartige Filtermaterial mit einem Protein behandelt worden ist.

7.  Diagnostische Vorrichtung nach Anspruch 1, worin das Flüssigkeitsabgabe-Element etwa 4 bis etwa 50 Rillen pro cm enthält, wobei die Rillen eine Tiefe von etwa 0,05 bis etwa 0,160 mm haben.

8.  Diagnostische Vorrichtung nach Anspruch 7, worin das Flüssigkeitsabgabe-Element ein im wesentlichen durchsichtiges polymeres Material darstellt.

9.  Diagnostische Vorrichtung nach Anspruch 8, worin das Flüssigkeitsabgabe-Element hydrolysiertes Celluloseacetat-Butyrat darstellt.

10. Diagnostische Vorrichtung nach Anspruch 1, enthaltend eine Vielzahl von Diagnosetest-Elementen.

**FIG 1**

10

**FIG 2**

**FIG 3**

FIG 4

FIG 5

FIG 6

FIG 7